# EUROPÄISCHE PATENTSCHRIFT

(11) **EP 1 158 962 B1**
(45) Veröffentlichungstag und Bekanntmachung des Hinweises auf die Patenterteilung: **04.12.2002**
(21) Anmeldenummer: 00916925.1
(22) Anmeldetag: 10.03.2000
(51) Int. Cl.: A61K 9/20, A61K 47/48

(54) **VERFAHREN ZUR HERSTELLUNG VON FESTEN CYCLODEXTRINHALTIGEN DOSIERUNGSFORMEN**
METHOD FOR PRODUCING SOLID DOSAGE FORMS CONTAINING CYCLODEXTRIN
PROCEDE DE FABRICATION DE FORMES DE DOSAGE SOLIDES RENFERMANT DE LA CYCLODEXTRINE

(30) Priorität: 12.03.1999 DE 19911097
(43) Veröffentlichungstag der Anmeldung: 05.12.2001
(73) Patentinhaber: BASF AKTIENGESELLSCHAFT, 67056 Ludwigshafen (DE)
(72) Erfinder: BERNDL, Gunther, D-67273 Herxheim (DE); REINHOLD, Ulrich, D-67346 Speyer (DE); SIMON, Dirk, D-67112 Mutterstadt (DE); LEHMANN, Stephan, D-67067 Ludwigshafen (DE)
(74) Vertreter: Kinzebach, Werner, Dr.
(86) Internationale Anmeldenummer: EP0002142
(87) Internationale Veröffentlichungsnummer: WO00054751

(56) Entgegenhaltungen:
- EP-A- 0 564 945
- WO-A-96/19962
- WO-A-97/18839
- GAZZANIGA A, ET AL.: "The use of .beta.-cyclodextrin as a pelletization agent in the extrusion/spheronization process" DRUG DEVELOPMENT AND INDUSTRIAL PHARMACY, Bd. 24, Nr. 9, September 1998 (1998-09), Seiten 869-873, XP000900430 ISSN: 0363-9045

## Beschreibung

Die vorliegende Erfindung betrifft ein Verfahren zur Herstellung von festen Dosierungsformen, enthaltend als Komponenten mindestens ein physiologisch verträgliches polymeres Bindemittel, mindestens einen Wirkstoff und mindestens ein Cyclodextrin.

Die Stoffgruppe der Cyclodextrine stellt wichtige Hilfsstoffe zur Fertigung von Nahrungsmitteln, Kosmetika, Pharmazeutika und Pestiziden dar und spielt auch bei einer Reihe von analytischen und technischen Trennproblemen eine wichtige Rolle. Ein Großteil der interessanten Eigenschaften der Cyclodextrine ist auf ihre Fähigkeit, mit Wirtsmolekülen sogenannte Gast-Wirt-Systeme zu bilden, zurückzuführen.

In pharmazeutischen Zusammensetzungen werden Cyclodextrine seit einiger Zeit verwendet, um die Eigenschaften der Dosierungsformen oder der darin eingesetzten Wirkstoffe zu modifizieren. Die beobachteten modifizierten Eigenschaften von Wirkstoffen in Gegenwart von Cyclodextrinen werden im Allgemeinen auf die Bildung von Cyclodextrin-Wirkstoffkomplexen zurückgeführt. Solche Cyclodextrin-Wirkstoffkomplexe zeigen oft verbesserte chemische Eigenschaften, wie z. B. erhöhte Stabilität gegenüber Licht- und Temperatureinwirkung, Oxidation, Reduktion, Hydrolyse und Dehydratisierung, und/oder geänderte physikalische Eigenschaften, wie z. B. Zustandsänderungen oder Änderungen der rheologischen Eigenschaften. Auch der Einfluß von Cyclodextrinen auf den Geschmack oder Geruch von Wirkstoffen ist bekannt.

Die Verarbeitung von Cyclodextrinen in Dosierungsformen kann auf mehrere Arten erfolgen. Zum einen kann das Cyclodextrin in kristalliner Form, z. B. als Pulver oder Granulat, dem Bindemittel und Wirkstoff zugemischt werden und anschließend, gegebenenfalls nach Homogenisation, zur Dosierungsform verpreßt werden, z. B. mittels gängiger Tablettierverfahren.

Zur Herstellung von Cyclodextrin-Wirkstoffkomplexen sind jedoch in der Regel zusätzliche Verfahrensschritte notwendig. Ein solches Verfahren ist die Komplexierung von Wirkstoffen mit Cyclodextrinen in wässriger Suspension oder Lösung. Nachteilig an diesem Verfahren ist, dass der erhaltene Cyclodextrin-Wirkstoff-Komplex aufgearbeitet werden muss, das heißt in der Regel abgetrennt und/oder getrocknet. Über eine beschleunigte Komplexierung von wirkstoffen in wässriger Suspension oder Lösung berichten T. Loftsson et al., Proc. Int. Symp. Cyclodextrines 8 (1996) 399-402 und Pharmazie 1998 (53) 11, 733-740, wenn bei der Komplexierung wasserlösliche Polymere zugegeben werden. Die Bildung eines ternären Intermediats wird postuliert.

Zum Beispiel offenbart die WO 98/55148 eine cyclodextrinhaltige pharmazeutische Zusammensetzung, bei der ein Wirkstoff, Citronensäure, Hydroxypropylcellulose und ein Cyclodextrin in Alkohol gelöst und der Alkohol anschließend abgedampft wird. Das erhaltene Gel wird in Gelatinekapseln gefüllt.

Um die Menge an eingesetzten Lösungsmitteln gering zu halten, empfehlen die WO 94/11031 und die FR-A-2705677 zur Herstellung von Cyclodextrin-Wirkstoff-Komplexen die Extrusion von lösungsmittelfeuchten Massen aus Cyclodextrin und Wirkstoff. Als geeignete Lösungsmittel werden unter anderem Wasser, Methanol oder Ethanol in Mengen von 20 bis 90 Gew.-%, bezogen auf die zu extrudierende Masse, genannt.

Die WO 97/18839 beschreibt ein Verfahren zur Herstellung fester Gemische aus Cyclodextrinen und Wirkstoffen durch Schmelzextrusion, wobei der Wirkstoff in den Cyclodextrinträger eingebettet wird. Das erhaltene Extrudat wird nach dem Erstarren vermahlen und unter Zusatz von Bindemitteln zur Herstellung von Dosierungsformen eingesetzt. Für dieses Verfahren sind Gehäusetemperaturen von mindestens 239 °C notwendig.

Seit einiger Zeit ist die Herstellung von Dosierungsformen durch Schmelzextrusion bekannt. Dabei wird kontinuierlich eine wirkstoffhaltige, in der Regel lösungsmittelfreie Schmelze aus einem polymeren wirkstoffhaltigen Bindemittel extrudiert und der extrudierte Strang zu der gewünschten Dosierungsform geformt, beispielsweise in einem Kalander mit Formwalzen oder Formbändern, siehe EP-A-240 904, EP-A-240 906 und EP-A-358 105.

Es besteht jedoch nach wie vor ein Bedarf an Dosierungsformen mit maßgeschneiderter Freisetzung des Wirkstoffs, insbesondere mit beschleunigter Wirkstofffreisetzung.

Der vorliegenden Erfindung lag daher die Aufgabe zugrunde, ein einfaches Verfahren zur Herstellung von festen Dosierungsformen mit beschleunigter Wirkstoff-Freisetzung zur Verfügung zu stellen.

Überraschenderweise wurde nun gefunden, dass man Dosierungsformen mit beschleunigter Wirkstoff-Freisetzung erhält, wenn man ein plastisches Gemisch aus Bindemittel und Wirkstoff in Gegenwart von Cyclodextrinen bildet und dieses zur Dosierungsform formt. Insbesondere war überraschend, dass man mit diesem Verfahren Dosierungsformen mit beschleunigter Freisetzung des Wirkstoffs erhält, ohne dass in vorgeschalteten Verfahrensschritten aufwendig ein Cyclodextrin-Wirkstoff-Komplex hergestellt werden muss.

Gegenstand der vorliegenden Erfindung ist daher ein Verfahren zur Herstellung von festen Dosierungsformen, enthaltend als Komponenten mindestens ein physiologisch verträgliches polymeres Bindemittel, mindestens einen Wirkstoff und mindestens ein Cyclodextrin, wobei man die Komponenten bei einer Temperatur unterhalb von 220 °C ohne Zugabe eines Lösungsmittels vermischt und plastifiziert und das erhaltene plastische Gemisch zur Dosierungsform formt.

Der Begriff "Dosierungsform" bezeichnet vorliegend eine beliebige Darreichungsform zur Verabreichung von Wirkstoffen an Mensch, Tier oder Pflanze. Die erfindungsgemäß erhaltenen Dosierungsformen sind insbesondere zur oralen oder rektalen Verabreichung oder als implantierbare Wirkstoffdepots bei Mensch und Tier geeignet. Besonders bevorzugte Dosierungsformen sind Tabletten jeglicher Form, Dragees, Pellets und Zäpfchen.

Geeignet für das erfindungsgemäße Verfahren sind polymere Bindemittel, die, dem jeweiligen Verwendungszweck der Dosierungsform angepasst, physiologisch verträglich sind. Vorzugsweise bilden die polymeren Bindemittel nach dem Erstarren eine Polymer-Matrix und sind in physiologischer Umgebung zumindest teilweise löslich oder quellbar. Beispiele für geeignete polymere Bindemittel sind:
Synthetische Polymere, wie Polyvinyllactame, insbesondere Polyvinylpyrrolidon (PVP), Copolymere von Vinyllactamen, wie N-vinylpyrrolidon, N-Vinylpiperidon und N-Vinyl-ε-caprolactam, aber insbesondere N-Vinylpyrrolidon, mit (Meth)acrylsäure, (Meth)acrylsäureestern, Vinylestern, insbesondere Vinylacetat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate, Polymethacrylate und Copolymerisate von Dimethylaminoethylacrylaten und Methacrylestern (z. B. Eudragit-Typen), Polyalkylenglykole, wie Polypropylenglykole und Polyethylenglykole, vorzugsweise mit Molekulargewichten oberhalb von 1 000, besonders bevorzugt oberhalb von 2 000 und ganz besonders bevorzugt oberhalb von 4 000 (z. B. Polyethylenglykol 600, Polyethylenglykol 6 000), Copolymerisate von Methylmethacrylat und Acrylsäure, Polyacrylamide, Polyvinylformamid (gegebenenfalls partiell oder vollständig hydrolysiert),
modifizierte natürliche Polymere, z. B. modifizierte Stärken und modifizierte Cellulosen, wie Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere HydroxypropylMethylcellulose oder Hydroxypropyl-Ethylcellulose, Cellulosephthalate, insbesondere Celluloseacetatphthalat und Hydroxypropylmethylcellulosephthalat, und
natürliche oder überwiegend natürliche Polymere, wie Gelatine, Polyhydroxyalkanoate, z. B. Polyhydroxybuttersäure und Polymilchsäure, Polyaminosäuren, z. B. Polylysin, Polyasparagin, Polydioxane und Polypeptide, und Mannane, insbesondere Galactomannane.

Hiervon sind die synthetischen und modifizierten natürlichen Polymere bevorzugt, insbesondere bevorzugt sind die synthetischen Polymere.

Bevorzugt als polymere Bindemittel sind davon Polyethylenglykol, Alkylcellulosen und Hydroxyalkylcellulosen, insbesondere Hydroxypropylcellulose und Hydroxypropylmethylcellulose, Polyhydroxyalkylacrylate und Polyhydroxyalkylmethacrylate, Polyacrylate und Polymethacrylate, Polyvinylpyrrolidon, Copolymerisate, enthaltend N-Vinyllactame, insbesondere N-Vinylpyrrolidon, und Vinylester, insbesondere Vinylacetat, Copolymerisate, enthaltend N-Vinyllactame, insbesondere N-Vinylpyrrolidon, und (Meth)acrylsäureester, und Gemische davon.

Besonders bevorzugt als polymere Bindemittel sind Polyethylenglykol, insbesondere mit Molekulargewichten oberhalb von 1 000 und bevorzugt oberhalb von 4 000, Polyvinylpyrrolidon, Copolymere, enthaltend N-Vinylpyrrolidon und Vinylacetat, und Gemische davon.

Vorteilhaft für die Verwendung als polymeres Bindemittel sind solche Bindemittel, die einen K-Wert (nach H. Fikentscher, Cellulose-Chemie 13 (1932), S. 58-64 und 71-74) im Bereich zwischen 10 und 100, insbesondere zwischen 15 und 80 aufweisen.

In besonderen Ausführungsformen des erfindungsgemäßen Verfahrens kann das Cyclodextrin teilweise oder vollständig chemisch an das polymere Bindemittel gebunden vorliegen.

Das plastische Gemisch weist in der Regel einen Gehalt an polymerem Bindemittel im Bereich von 5 bis 99,8 Gew.-%, vorzugsweise von 10 bis 98 Gew.-% und besonders bevorzugt von 15 bis 80 Gew.-% auf.

Unter Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht wesentlich zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,01 bis 60 Gew.-%, vorzugsweise 0,5 bis 30 Gew.-%, und insbesondere 1 bis 25 Gew.-% liegen. Auch Wirkstoff-Kombinationen können eingesetzt werden. Wirkstoffe im Sinne der Erfindung sind auch Vitamine, Mineralstoffe und Pflanzenauszüge oder Pflanzenzubereitungen mit pharmazeutisch wirksamen Bestandteilen sowie Pflanzenbehandlungsmittel und Insektizide. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu den Pflanzenauszügen oder Pflanzenzubereitungen zählen z. B. Pflanzentrockenextrakte, insbesondere Johanniskrautextrakt, Mariendistelextrakt, Kava-Kava-Extrakt, Schöllkrautextrakt, Gingko Biloba-Extrakt, und etherische Öle, insbesondere Knoblauchöl, Kamillenöl, Pfefferminzöl, Kümmelöl und Eukalyptusöl. Zu Wirkstoffen im Sinne der Erfindung gehören auch Peptidtherapeutika und Impfstoffe.

Das erfindungsgemäße Verfahren ist beispielsweise zur Verarbeitung folgender Wirkstoffe beziehungsweise der pharmakologisch aktiven Salze davon geeignet:
Acebutolol, Acetylcystein, Acetylsalicylsäure, Acyclovir, Alfacalcidol, Allantoin, Allopurinol, Alprazolam, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benexat, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefadroxil, Cefalexin, Cefazolin, Cefixim, Cefotaxim, Cefotiam, Ceftazidim, Ceftriaxon, Cefuroxim, Chloramphenicol, Chlordiazepoxid, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Digitoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Folinsäure, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Imipramin, Indomethacin, Iohexol, Iopamidol, Isosorbid-Dinitrat, Isosorbid-Mononitrat, Isotretinoin, Itraconazol, Iod, Ketotifen, Ketoconazol, Ketoprofen, Ketorolac, Knoblauchöl, Labetalol, Lactulose, Lecithin, Levocarnitin, Levodopa, Levoglutamid, Levonorgestrel, Levothyroxin, Lidocain, Lipase, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nitrogycerin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Pentoxifyllin, Phenobarbital, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Propafenon, Propranolol, Prostaglandine, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Selegilin, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Tiaprofensäure, Uracil, Valproinsäure, Vancomycin, Verapamil, Vitamin E, Zidovudin.

Bevorzugte Wirkstoffe sind Ibuprofen (als Racemat, Enantiomer oder angereichertes Enantiomer), Ketoprofen, Flurbiprofen, Benexat, Cefotiam, Chlordiazepoxid, Digitoxin, Tiaprofensäure, Knoblauchöl, Diazepam, Piroxicam, Itraconazol, Ketoconazol, Iod, Nystatin, Prostaglandine, Hydrocortison, Dexamethason, Estradiol, Verapamil, Nifedipin, Nitroglycerin, Omeprazol, Ranitidin, Cyclosporin, Trandolapril und Peptidtherapeutika.

Cyclodextrine im Sinne der Erfindung sind cyclische Oligo- oder Polyzucker, z. B. sogenannte Cycloamylosen oder Cycloglucane, und analoge cyclische Kohlenhydrate, die z. B. in Angew. Chem. 92 (1980) S. 343 oder F. Vögtle, Supramolekulare Chemie, 2.Aufl., (1992) beschrieben sind. Geeignet sind vorzugsweise Cyclodextrine, die eine geeignete Struktur für Wechselwirkungen mit Wirkstoffmolekülen, insbesondere im Sinne von Wirt-Gast-Systemen, aufweisen. Insbesondere sind solche Cyclodextrine geeignet, die aus 6, 7, 8 oder 9 α-1,4-glycosidisch verknüpften Glucoseeinheiten bestehen, die sogenannten α-, β-, γ- oder δ-Cyclodextrine. Es sind auch höhere cyclodextrinanaloge Strukturen, die aus einer größeren Anzahl von Glucosen oder ähnlichen Zuckern aufgebaut sind, denkbar und geeignet.

Ebenfalls geeignet als Cyclodextrine sind modifizierte Cyclodextrine, wie z. B. die Reaktionsprodukte, die durch Umsetzung von Cyclodextrinen mit Alkylenoxiden, Alkylhalogeniden, Säurechloriden, Epihalohydrinen, Isocyanaten oder halogenierten Carbonsäuren herstellbar sind. So eignen sich beispielsweise Umsetzungsprodukte von Cyclodextrinen mit Alkylenoxiden, wie Ethylenoxid, Propylenoxid, Butylenoxid oder Styroloxid. In den so gebildeten Cyclodextrin-Polyethern können eine, mehrere oder alle Hydroxygruppen substituiert sein. In Abhängigkeit vom Substitutionsgrad oder den Kettenlängen der Polyethereinheiten liegt der mittlere molare Substitutionsgrad, d. h. die Anzahl der Mole Alkylenoxid, mit denen ein Mol Cyclodextrin umgesetzt wird, in der Regel zwischen 3 und 20 000, ist aber prinzipiell nicht nach oben beschränkt. Besonders geeignet sind beispielsweise die Umsetzungsprodukte von Cyclodextrinen mit Alkylierungsmitteln, wie C₁- bis C₂₂-Alkylhalogeniden, z. B. Methylchlorid, Ethylchlorid, Isopropylchlorid, n-Butylchlorid, Isobutylchlorid, Benzylchlorid, Laurylchlorid, Stearylchlorid, Methylbromid, Ethylbromid, n-Butylbromid, und Dialkylsulfaten, wie z. B. Dimethylsulfat oder Diethylsulfat. Die Umsetzung mit Alkylierungsreagenzien führt zu Cyclodextrinethern, in denen eine, mehrere oder alle Hydroxygruppen durch Alkylethergruppen substituiert sind. Bei aus Glucoseeinheiten aufgebauten Cyclodextrinen liegt der durchschnittliche Veretherungsgrad pro Glucoseeinheit in der Regel im Bereich von 0,5 bis 3, bevorzugt im Bereich von 0,1 bis 2,5 und besonders bevorzugt im Bereich von 1 bis 2. Insbesondere bevorzugt sind methylierte, ethylierte oder propylierte α-, β- oder γ-Cyclodextrine mit einem mittleren Veretherungsgrad von 1,5 bis 2,2. Geeignet sind auch Cyclodextrinester, wie sie durch Umsetzung von Cyclodextrinen mit Säurechloriden, wie Carbonsäure- oder Sulfonsäurechloriden, erhältlich sind. Insbesondere geeignet sind Carbonsäurechloride, wie Acetylchlorid, Acrylsäurechlorid, Methacrylsäurechlorid oder Benzoylchlorid.

Ebenfalls geeignet sind polymermodifizierte Cyclodextrine, d. h. Cyclodextrine, die in die Hauptkette von Polymeren eingebaut sind und/oder Cyclodextrine, die an Seitenketten von Polymeren angelagert sind oder selbst Seitenketten von Polymeren darstellen. Polymermodifizierte Cyclodextrine, bei denen die enthaltenen Cyclodextrineinheiten in der Hauptkette des Polymers angeordnet sind, sind z.B. durch Umsetzung von Cyclodextrinen mit oder in Gegenwart von geeigneten Kupplungs- oder Vernetzungsreagenzien zugänglich, z. B. wie in Helv. Chim. Acta, Bd. 48, (1965), S. 1225, beschrieben. Polymermodifizierte Cyclodextrine, bei denen die Cyclodextrineinheiten Seitenkettenbestandteile sind oder als Seitenketten fungieren, sind z. B. durch Polymerisation von mit polymerisierbaren Gruppen modifizierten Cyclodextrinen mit weiteren Comonomeren zugänglich, z. B. durch Polymerisation von Cyclodextrin(meth)acrylaten in Gegenwart weiterer ethylenisch ungesättigter Monomere oder durch radikalisches Pfropfen von Cyclodextrin(meth)acrylaten auf Polymere mit freien Hydroxygruppen, wie z. B. Polyvinylalkohol. Eine weitere Möglichkeit zur Herstellung polymermodifizierter Cyclodextrine mit den Cyclodextrineinheiten an Seitengruppen oder als Seitengruppen von Polymeren stellt die Umsetzung von Cyclodextrinen, deprotonierten Cyclodextrinen oder ihren Alkalisalzen mit Polymeren, die komplementäre reaktive Gruppen aufweisen, wie z. B. Anhydrid-, Isocyanat-, Säurehalogenid- oder Epoxygruppen oder Halogene, dar.

Weitere geeignete polymermodifizierte Cyclodextrine und Verfahren zu ihrer Herstellung sind in der DE-A-196 12 768 beschrieben.

Es können auch solche polymermodifizierten Cyclodextrine eingesetzt werden, bei denen der Polymeranteil der Polymermodifikation bereits das polymere Bindemittel darstellt.

Dies ist insbesondere dann der Fall, wenn das Molekulargewicht solcher polymermodifizierten Cyclodextrine größer als 10 000, bevorzugt größer als 20 000 oder 50 000 ist. In diesen Fällen liegt das Cyclodextrin in chemisch an das polymere Bindemittel gebundener Form vor.

Bei polymermodifizierten Cyclodextrinen bzw. chemisch an das polymere Bindemittel gebundenen Cyclodextrinen mit Molekulargewichten von größer als 30 000 ist der Polymeranteil dem Gesamtpolymergehalt des plastischen Gemischs zuzurechnen. Bei polymermodifizierten Cyclodextrinen bzw. chemisch an das polymere Bindemittel gebundenen Cyclodextrinen mit Molekulargewichten von größer als 30 000 ist der Cyclodextrinanteil dem Gesamtcyclodextringehalt des plastischen Gemisches zuzurechnen. Beispiele für geeignete chemisch an das polymere Bindemittel gebundene Cyclodextrine sind entsprechend hochmolekulare polyalkylenethermodifizierte Cyclodextrine oder Cyclodextrin(meth)acrylat-Copolymere mit (Meth)acrylsäure, (Meth)acrylsäureestern, Vinylacetat und/oder N-Vinylpyrrolidon als Comonomere.

Das plastische Gemisch kann 0,1 bis 90 Gew.-%, vorzugsweise 0,5 bis 70 Gew.-% und besonders bevorzugt 1 bis 60 Gew.-% mindestens eines Cyclodextrins enthalten. Vorzugsweise wird die Menge an Cyclodextrin so gewählt, dass das Molverhältnis zwischen Wirkstoff und Cyclodextrin im Bereich von 0,1 bis 4,0, bevorzugt im Bereich von 0,5 bis 3 und besonders bevorzugt im Bereich von 0,8 bis 2,0 liegt. In bevorzugten Ausführungsformen des erfindungsgemäßen Verfahrens hat es sich als vorteilhaft erwiesen, Wirkstoff und Cyclodextrin in ungefähr äquimolaren Mengen einzusetzen.

Darüber hinaus können in dem erfindungsgemäßen Verfahren Hilfsstoffe eingesetzt werden, z. B. solche, die die Herstellung der Dosierungsformen erleichtern und/oder die Eigenschaften der erhaltenen Dosierungsformen verbessern.

Solche Hilfsstoffe sind z. B. übliche galenische Hilfsstoffe, deren Gesamtmenge bis zu 100 Gew.-%, bezogen auf das polymere Bindemittel, betragen kann, z. B. Streckmittel bzw. Füllstoffe, wie Silikate oder Kieselerde, Magnesiumoxid, Aluminiumoxid, Titanoxid, Methylcellulose, Natrium-Carboxymethylcellulose, Zuckeralkohole, wie z. B. Mannit, Sorbit, Xylit und Isomalt, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches;
Schmiermittel und Trennmittel wie Magnesium-, Aluminium- und Calciumstearat, Talkum und Silicone, sowie tierische oder pflanzliche Fette, insbesondere in hydrierter Form und solche, die bei Raumtemperatur fest sind. Diese Fette haben vorzugsweise einen Schmelzpunkt von 30 °C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Monound Diglyceride stammen vorzugsweise von den oben erwähnten Fettsäuretypen ab. Die Gesamtmenge an Schmier- und Trennmitteln beträgt vorzugsweise 0,1 bis 10 Gew.-%, bezogen auf das Gesamtgewicht der Masse für die jeweilige Schicht;
Fließmittel, z.B, Aerosil, in einer Menge von 0,1 bis 5 Gew.-%, bezogen auf das Gesamtgewicht des Gemisches;
Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;
Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions- und Formentrennmittel sowie Tenside, vorzugsweise anionische und nicht-ionische, wie z. B. Seifen und seifenähnliche Tenside, Alkylsulfate und -sulfonate, Salze von Gallensäuren, alkoxyilierte Fettalkohole, alkoxylierte Alkylphenole, alkoxylierte Fettsäuren und Fettsäureglycerinester, die alkoxyliert sein können, und Solubilisierungsmittel, wie Cremophor (polyethoxyliertes Ricinusöl), Gelosire, Vitamin E-TPGS und Tween (ethoxylierte Sorbitanfettsäureester), zugesetzt werden (vgl. z. B. H. Sucker et al. Pharmazeutische Technologie, Thieme-Verlag, Stuttgart 1978).

Unter Hilfsstoffen im Sinne der Erfindung sind auch Substanzen zur Herstellung einer festen Lösung mit dem pharmazeutischen Wirkstoff zu verstehen. Diese Hilfsstoffe sind beispielsweise Pentaerythrit und Pentaerythrittetraacetat, Harnstoff, Phosphatide, wie Lecithin, Polymere, wie z. B. Polyethylen- bzw. Polypropylenoxide und deren Blockcopolymere (Poloxamere), Homo- und Copolymere des Vinylpyrrolidons, insbesondere Copolymere von Vinylpyrrolidon und Vinylacetat, Poly(meth)acrylate (z. B. Eudragit-Typen), Tenside wie Polyoxyethylen-40-stearat sowie Zitronen- und Bernsteinsäure, Gallensäuren, Sterine und andere, wie z. B. bei J. L. Ford, Pharm. Acta Helv. 61, (1986), S. 69-88, angegeben.

Als pharmazeutische Hilfsstoffe gelten auch Zusätze von Basen und Säuren zur Steuerung der Löslichkeit eines Wirkstoffes (siehe beispielsweise K. Thoma et al., Pharm. Ind. 51, (1989), S. 98-101).

Die einzigen Voraussetzungen für die Eignung von Hilfsstoffen sind ausreichende Temperaturstabilität und die Kompatibilität mit dem verwendeten Wirkstoff.

Für die Bildung des plastischen Gemischs ist es erforderlich, die Bestandteile, nämlich mindestens ein thermoplastisches, physiologisch verträgliches Bindemittel und mindestens einen Wirkstoff und mindestens ein Cyclodextrin zu vermischen und in den plastischen Zustand zu überführen. Vorzugsweise erfolgt die Bildung des plastischen Gemisches ohne Zusatz eines Lösungsmittels. Lösungsmittel im Sinne der Erfindung sind niedermolekulare flüchtige Flüssigkeiten, z. B. Wasser, C₁- bis C₆-Monoalkohole und deren Ether, Ester von C₁- bis C₆-Monoalkanolen mit C₁- bis C₆-Carbonsäuren, Alkane, Aromaten und substituierte Aromaten mit bis zu 12 Kohlenstoffatomen und chlorierte Kohlenwasserstoffe, wie z. B. Methylenchlorid. Ein weiteres brauchbares Lösungsmittel ist flüssiges CO₂.

In bestimmten Fällen kann es vorteilhaft sein, Wirkstoffe und/oder Hilfsstoffe als Lösung oder Suspension in einem Lösungsmittel, z. B. einem der vorgenannten Lösungsmittel, bei der Bildung des plastischen Gemisches zuzugeben. Beispielsweise kommen pharmazeutische Wirkstoffe häufig in Form eines Salzes, das im Allgemeinen wasserlöslich ist, zur Anwendung. Wasserlösliche Wirkstoffe können daher als wässrige Lösung eingesetzt werden oder vorzugsweise in die wässrige Lösung oder Dispersion des Bindemittels aufgenommen werden. Entsprechendes gilt für Wirkstoffe, die in einem der genannten Lösungsmittel löslich sind, wenn die flüssige Form der zur Anwendung kommenden Komponenten auf einem organischen Lösungsmittel basiert. Die erfindungsgemäß einzusetzenden Komponenten können geringe Mengen der vorgenannten Lösungsmittel enthalten, z. B. aufgrund von Hygroskopie, Lösungsmitteleinschlüssen oder Kristallwasser. Der Gesamtlösungsmittelgehalt des plastischen Gemisches liegt vorzugsweise unter 15 %, insbesondere unter 10 % und besonders bevorzugt unter 5 %.

Die Bildung des plastischen Gemisches kann durch Aufschmelzen oder auch durch Kneten, Vermischen oder Homogenisieren unterhalb der Schmelztemperatur des Bindemittels durchgeführt werden. Vorzugsweise bildet man das plastische Gemisch bei Temperaturen un-terhalb von 220 °C. Vorzugsweise erfolgt die Bildung des plastischen Gemischs nicht durch Anteigen oder partielles Lösen einer oder mehrerer Komponenten (d. h. Bindemittel, Wirkstoff und/oder Cyclodextrin) mit Flüssigkeiten oder Lösungsmitteln, wie z. B. die oben genannten, sondern hauptsächlich oder ausschließlich durch thermische oder thermisch-mechanische Einwirkung auf die Komponente(n) (d. h. durch thermisches Plastifizieren). Bevorzugt erfolgt die Bildung des plastischen Gemischs durch Extrusion, besonders bevorzugt durch Schmelzextrusion. Die Verfahrensschritte des Plastifizierens können auf an sich bekannte Art und Weise durchgeführt werden, beispielsweise wie in der EP-A-0 240 904, EP-A-0 337 256, EP-A-0358 108, WO 97/15290 und WO 97/15291 beschrieben.

Die Komponenten, d. h. Bindemittel, Wirkstoff und Cyclodextrin und gegebenenfalls Hilfsstoffe, können zuerst vermischt und dann in den plastischen Zustand überführt und homogenisiert werden. Insbesondere bei Verwendung von empfindlichen Wirkstoffen hat es sich aber als bevorzugt erwiesen, zuerst das polymere Bindemittel und das Cyclodextrin, gegebenenfalls zusammen mit üblichen pharmazeutischen Additiven in den plastischen Zustand überzuführen und vorzuvermischen, wobei die Apparaturen, wie Rührkessel, Rührwerke, Feststoffmischer etc., gegebenenfalls im Wechsel betrieben werden und dann den (die) empfindlichen Wirkstoff(e) in "Intensivmischern" in plastischer Phase bei sehr kleinen Verweilzeiten einzumischen (homogenisieren). Der (die) Wirkstoff(e) kann (können) in fester Form oder als Lösung, Suspension oder Dispersion eingesetzt werden.

In bestimmten Ausführungsformen des erfindungsgemäßen Verfahrens kann es vorteilhaft sein, erst ein plastisches Gemisch aus Bindemittel und Wirkstoff und gegebenenfalls Hilfsstoffen zu bilden und zu diesem das Cyclodextrin zu geben. Diese Vorgehensweise kann insbesondere dann vorteilhaft sein, wenn der Wirkstoff weichmacherähnliche Eigenschaften besitzt und die dadurch erreichbare Erniedrigung der Gesamtverfahrenstemperatur wünschenswert ist.

In bestimmten Ausführungsformen des erfindungsgemäßen Verfahrens kann es vorteilhaft sein, erst Wirkstoff und Cyclodextrin zu vermischen und zu dem plastifizierten Bindemittel zu geben. Diese Vorgehensweise kann insbesondere dann vorteilhaft sein, wenn der Wirkstoff und/oder das Cyclodextrin thermisch labil ist (sind).

Das plastifizieren, Aufschmelzen und/oder Vermischen erfolgt in einer für diesen Zweck üblichen Vorrichtung. Besonders geeignet sind Extruder oder beheizbare Behälter mit Rührwerk, z. B. Kneter, (wie der unten noch erwähnten Art).

Als Mischapparat sind auch solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z. B. Dispax der Firma IKA).

Bei empfindlichen Wirkstoffen erfolgt vorzugsweise zunächst das Überführen des polymeren Bindemittels und des Cyclodextrins in den plastischen Zustand in einem Extruder und anschließend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Das Beschicken der Mischvorrichtung erfolgt je nach deren Konzeption kontinuierlich oder diskontinuierlich in üblicher Weise. Pulverförmige Komponenten können im freien Zulauf, z. B. über eine Differentialdosierwaage eingeführt werden. Plastische Massen können direkt aus einem Extruder eingespeist oder über eine Zahnradpumpe, die insbesondere bei hohen Viskositäten und hohen Drükken von Vorteil ist, zugespeist werden. Flüssige Medien können über ein geeignetes Pumpenaggregat zudosiert werden.

Das durch Vermischen und Überführen des Bindemittels, gegebenenfalls des Wirkstoffes und/oder Cyclodextrins und gegebenenfalls des Additivs oder der Additive, in den plastischen Zustand erhaltene Gemisch ist teigig, zähflüssig oder dünnflüssig (thermoplastisch) und daher auch extrudierbar. Die Glasübergangstemperatur des Gemisches liegt vorzugsweise unter der Zersetzungstemperatur aller in dem Gemisch enthaltenen Komponenten.

Die Verfahrensschritte Vermischen und Aufschmelzen können in derselben Apparatur oder in zwei oder mehreren getrennt arbeitenden Vorrichtungen ausgeführt werden. Die Zubereitung einer Vormischung kann in einer der oben beschriebenen üblichen Mischvorrichtungen durchgeführt werden. Eine solche Vormischung kann dann direkt, z. B. in einen Extruder, eingespeist und anschließend gegebenenfalls unter Zusatz weiterer Komponenten extrudiert werden.

Das erfindungsgemäße Verfahren erlaubt es, als Extruder Einschneckenmaschinen, kämmende Schneckenmaschinen oder auch Mehrwellextruder, insbesondere Zweischnecken-Extruder, gleichsinnig oder gegensinnig drehend und gegebenenfalls mit Knetscheiben ausgerüstet, einzusetzen. Wenn bei der Extrusion ein Lösungsmittel verdampft werden muss, sind die Extruder im Allgemeinen mit einem Verdampfungsteil ausgerüstet. Besonders bevorzugt sind Extruder der ZKS-Baureihe von Werner u. Pfleiderer.

Mit dem erfindungsgemäßen Verfahren können auch mehrschichtige cyclodextrinhaltige Dosierungsformen durch Koextrusion hergestellt werden, wobei mehrere Gemische aus den oben beschriebenen Komponenten, von denen mindestens eins Cyclodextrin(e) enthält, bei der Extrusion so in einem Werkzeug zusammengeführt werden, dass sich der gewünschte Schichtaufbau der mehrschichtigen Dosierungsform ergibt. Vorzugsweise verwendet man verschiedene Bindemittel für verschiedene Schichten.

Mehrschichtige Dosierungsformen umfassen vorzugsweise zwei oder drei Schichten. Sie können in offener oder geschlossener Form vorliegen, insbesondere als offene oder geschlossene Mehrschichttabletten.

Das Ausformen erfolgt durch Koextrusion, wobei die Gemische aus den einzelnen Extrudern oder anderen Aggregaten in ein gemeinsames Koextrusionswerkzeug geführt und ausgetragen werden. Die Form der Koextrusionswerkzeuge richtet sich nach der gewünschten Dosierungsform. Beispielsweise sind Werkzeuge mit ebenem Austrittsspalt, sogenannte Breitschlitzwerkzeuge, und Werkzeuge mit kreisringspaltförmigem Austrittsquerschnitt geeignet. Die Düsenauslegung erfolgt dabei in Abhängigkeit von dem zur Anwendung kommenden polymeren Bindemittel und der gewünschten Dosierungsform.

Das polymere Bindemittel muss sich in der Gesamtmischung aller Komponenten im Bereich von 30 bis 200 °C, vorzugsweise 40 bis 170 °C in einen plastischen Zustand überführen lassen. Die Glasübergangstemperatur der Mischung muss daher unter 220 °C, vorzugsweise unter 180 °C liegen. Erforderlichenfalls wird sie durch übliche, pharmakologisch akzeptable weichmachende Hilfsstoffe herabgesetzt. Die Menge an Weichmacher beträgt höchstens 30 Gew.-%, bezogen auf das Gesamtgewicht von Bindemittel und Weichmacher, damit lagerstabile Arzneiformen gebildet werden, die keinen kalten Fluss zeigen. Vorzugsweise aber enthält das Gemisch keinen Weichmacher.

Beispiele für derartige Weichmacher sind:
organische, vorzugsweise schwerflüchtige Verbindungen, wie z. B. C₇- bis C₃₀-Alkanole, Ethylenglykol, Propylenglykol, Glycerin, Trimethylolpropan, Triethylenglykol, Butandiole, Pentanole, wie Pentaerythrit und Hexanole, Polyalkylenglykole, vorzugsweise mit einem Molekulargewicht von 200 bis 1 000, wie z. B. Polyethylenglykole, Polypropylenglykole und Polyethylenpropylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im Allgemeinen 0,5 bis 15, vorzugsweise 0,5 bis 5 Gew.-%, bezogen auf das Gesamtgewicht des plastischen Gemisches.

In bevorzugten Ausführungsformen der vorliegenden Erfindung setzt man zur Herstellung des plastischen Gemischs
a) 0,5 bis 30 Gew.-% mindestens eines Wirkstoffs,
b) 0,5 bis 70 Gew.-% mindestens eines Cyclodextrins,
c) 10 bis 98 Gew.-% mindestens eines polymeren Bindemittels und
d) 0 bis 50 Gew.-% übliche Hilfsmittel ein.

Das erfindungsgemäße Verfahren kann vorteilhaft ganz oder teilweise unter sterilen Arbeitsbedingungen durchgeführt werden, z. B. in Reinräumen und unter Verwendung sterilisierter Geräte, wie z. B. Waagen, Mischern, Extrudern und Formungsmaschinen, wie Kalandern, Quetschvorrichtungen und Zerhackern. Die Einsatzstoffe können entweder in sterilisierter Form, gegebenenfalls unter Zugabe geeigneter antibakterieller und/oder antiviraler Hilfsstoffe, in das Verfahren eingebracht werden und/oder die Verfahrensbedingungen, insbesondere die Temperatur, so gewählt werden, dass sterile erfindungsgemäße Dosierungsformen erhalten werden. Die erhaltenen sterilen Dosierungsformen können anschließend vorteilhaft unter ebenfalls sterilen Bedingungen weiterverarbeitet werden, z. B. zu parenteralen Präparaten, oder direkt Verpackt werden, z. B. durch Verblistern oder Einschweißen. Die Formgebung und das Verpacken kann auch gleichzeitig durchgeführt werden, insbesondere wenn die Formgebung des plastischen Gemischs durch Kalandrieren mittels Formwalzen durchgeführt wird. Dazu bringt man zusätzlich zu dem plastischen Gemisch als Folien vorliegende Materialien jeweils zwischen Schmelze und Formwalze, wodurch gleichzeitig mit der Formung des plastischen Gemischs zu Dosierungsformen eine Umhüllung und/oder eine Verpackung der Dosierungsform erreicht werden kann, wie in der WO-96/19963 beschrieben, auf die hiermit Bezug genommen wird.

Im Einzelnen kann es bei dem erfindungsgemäßen Verfahren zur Ausbildung von festen Lösungen kommen. Der Begriff "feste Lösungen" ist dem Fachmann geläufig, beispielsweise aus der eingangs zitierten Literatur. In festen Lösungen von Cyclodextrin-Wirkstoff-Komplexen oder Wirkstoffen und/oder Cyclodextrinen in Polymeren liegen Cyclodextrin-Wirkstoff-Komplex oder Wirkstoff und/oder Cyclodextrin molekulardispers im Polymer vor.

Ein weiterer Vorteil der vorliegenden Erfindung ist, dass mit diesem Verfahren feste Dosierungsformen herstellbar sind, in denen der (die) Wirkstoff(e) mit dem (den) Cyclodextrin(en) wechselwirken, z. B. in Form einer Bildung von Cyclodextrin-Wirkstoff-Komplexen. Bevorzugte Cyclodextrin-Wirkstoff-Komplexe sind sogenannte Einschluss- oder Wirt-Gast-Komplexe. Die Bildung solcher Komplexe kann durch Einlagerung von Wirkstoffen in Kavitäten von Cyclodextrinaggregaten, z. B. durch sogenannte molekulare Verkapselung oder durch stöchiometrische oder angenähert stöchiometrische Wechselwirkung von einem oder mehreren Wirkstoffmolekülen mit einem oder mehreren Cyclodextrinmolekülen erfolgen.

Die erfindungsgemäßen Dosierungsformen zeigen bessere Freisetzungseigenschaften, erhöhte Stabilität der enthaltenen Wirk- und Hilfsstoffe und/oder verbesserte sensorische Eigenschaften, z. B. Aussehen, Geruch und/oder Geschmack.

Das erfindungsgemäße Verfahren kann vorteilhaft bei Temperaturen unterhalb von 200 °C und bevorzugt unterhalb von 170 °C, aber oberhalb von Raumtemperatur (25 °C), vorzugsweise oberhalb von 40 °C, durchgeführt werden. Insbesondere führt man das Verfahren in einem Temperaturintervall durch, das sich 40 °C, bevorzugt 30 °C und besonders bevorzugt 20 °C von der Erweichungstemperatur des Gemischs der Komponenten beziehungsweise den Schmelzpunkt der zuerst plastifizierten Hauptkomponente(n) nach oben oder unten erstreckt. Insbesondere vorteilhaft ist, dass mit dem erfindungsgemäßen Verfahren bei den vorgenannten Temperaturen Cyclodextrin-Wirkstoff-Komplexe beziehungsweise Cyclodextrin-Wirkstoff-Komplexhaltige feste Dosierungsformen entstehen können.

In den mit dem erfindungsgemäßen Verfahren herstellbaren Dosierungsformen können 0 bis 100 % , vorzugsweise 0,1 bis 99,5 % und insbesondere 5 bis 99 % des oder der Wirkstoffe als Cyclodextrin-Wirkstoff-Komplex vorliegen. In einer bevorzugten Ausführungsform des erfindungsgemäßen Verfahrens liegen wenigstens 10 %, insbesondere wenigstens 30 % des eingesetzten Wirkstoffs in Form eines Cyclodextrin-Wirkstoff-Komplexes vor. In einer weiteren bevorzugten Ausführungsform des erfindungsgemäßen Verfahrens liegt der Wirkstoff im Wesentlichen vollständig in Form eines Cyclodextrin-Wirkstoff-Komplexes vor.

Das erfindungsgemäße plastische Gemisch eignet sich, gegebenenfalls nach dem Abkühlen oder Erstarren, für alle gängigen Verfahren zur Herstellung fester Dosierungsformen, wie Granulieren, Mahlen, Pressen, Formgießen, Spritzgießen, Tablettieren unter Druck, Tablettieren unter Druck und Wärme und insbesondere für die Extrusion beziehungsweise Schmelzextrusion.

Mit dem erfindungsgemäßen Verfahren lassen sich eine Vielzahl von festen Dosierungsformen formen. So lassen sich z. B. durch Mahlen oder Zerhacken des erstarrten oder zumindest teilweise erstarrten plastischen Gemischs Pulver oder Granulate herstellen, die entweder direkt zur Therapierung eingesetzt oder gegebenenfalls unter Zugabe üblicher Hilfsstoffe, zu Pellets, Tabletten, Zäpfchen, Implantaten und parenteralen Präparaten weiterverarbeitet werden können.

Vorzugsweise werden mit dem erfindungsgemäßen Verfahren Dosierungsformen vor dem Erstarren des plastischen Gemischs geformt, die, gegebenenfalls nach Beschichten, Coaten, Dragieren oder mit einem Film überziehen, in therapeutisch einsetzbarer Form anfallen.

Die Formung zur Dosierungsform vor dem Erstarren kann in Abhängigkeit von der Viskosität des plastischen Gemischs auf vielfältige Weise erfolgen, z. B. durch Formgießen, Spritzgießen, Pressen, Quetschen oder Kalandrieren. Dazu wird das vorstehend beschriebene plastische Gemisch im erfindungsgemäßen Verfahren einem oder mehreren Formungsschritten zugeführt. Das Zuführen kann durch Pressen, Pumpen, z. B. mit Zahnradpumpen, oder vorzugsweise mit einem Extruder erfolgen.

Besonders bevorzugt wird das plastische Gemisch in einem oder mehreren, bevorzugt einem, Extruder gebildet und mit diesem oder einem nachgeschalteten Extruder den Formungsschritten zugeführt. In vielen Fällen hat es sich als vorteilhaft herausgestellt, schräg abwärts zu extrudieren und/oder gegebenenfalls eine Führungsrinne zum Transport des Extrudats vorzusehen, um einen sicheren Transport zu gewährleisten und ein Abreißen des extrudierten Stranges zu verhindern. In Abhängigkeit von der Anzahl und Verträglichkeit der einzusetzenden Wirkstoffe und/oder Cyclodextrin-Wirkstoffkomplexe können vorteilhaft auch mehrschichtige Extrudate, z. B. Coextrudate, wie in der WO 96/19963 beschrieben, bei dem erfindungsgemäßen Verfahren eingesetzt werden.

Der erste Formungsschritt erfolgt vorteilhaft beim Austrag des Extrudats aus dem Extruder durch geeignet geformte Düsen, Blenden oder sonstige Austrittsöffnungen, z. B. durch eine Lochblende, eine Runddüse oder eine Breitschlitzdüse. In der Regel wird so kontinuierlich ein Strangextrudat mit vorzugsweise konstantem Querschnitt, z. B. in Form eines Bandes oder eines Stranges, vorzugsweise mit rundem, ovalem, abgerundetem oder flachem und breitem Querschnitt, erhalten.

Geeignete nachgeschaltete Formungsschritte für Extrudate sind z. B. der Kaltabschlag, d. h. das das Schneiden beziehungsweise Zerhacken des Stranges nach zumindest teilweisem Erstarren, der Heißabschlag, d. h. das Zerschneiden beziehungsweise Zerhacken des. Stranges in noch plastischer Form oder das Abquetschen des noch plastischen Strangs in einer Quetschvorrichtung. Mit Heiß- oder Kaltabschlag lassen sich z. B. Granulate (Heiß- oder Kaltgranulierung) oder Pellets erhalten. Die Heißgranulierung führt in der Regel zu Dosierungsformen (Tabletten oder Pellets) mit einem Durchmesser von 0,1 bis 10 mm, während die Kaltgranulierung normalerweise zu zylinderförmigen Produkten mit einem Verhältnis von Länge zu Durchmesser von 1 bis 10 und einem Durchmesser von 0,5 bis 10 mm führt. So können einschichtige, bei Anwendung der Koextrusion aber auch offene oder geschlossene mehrschichtige Dosierungsformen hergestellt werden, beispielsweise Oblongtabletten, Dragees, Pastillen und Pellets. Die Dosierungsformen können in einem nachgeschalteten Verfahrensschritt nach üblichen Methoden mit einem Coating versehen werden. Geeignete Materialien für Filmüberzüge sind die als polymere Bindemittel genannten Polymere, insbesondere Polyacrylate, wie die Eudragit-Typen, Celluloseester, wie die Hydroxypropylcellulosephthalate, sowie Celluloseether, wie Ethylcellulose, Hydroxypropylmethylcellulose oder Hydroxypropylcellulose und Gelatine. Auch weitere Formungsschritte können sich anschließen, wie z. B. die Arrondierung oder Verrundung der durch den Heiß- oder Kaltabschlag erhaltenen Pellets mittels Arrondiervorrichtungen, wie in der DE-A-196 29 753 beschrieben.

Besonders bevorzugt werden alle Formungsschritte am noch plastischen Gemisch oder noch plastischen Extrudat durchgeführt. Neben dem Heißabschlag, gegebenenfalls mit nachfolgendem Arrondieren, eignet sich insbesondere ein Verfahren zur Herstellung der festen Dosierungsformen, bei dem man das plastische Gemisch in einem Formkalander zur Dosierungsform formt. Dazu wird ein noch plastisches Gemisch oder ein noch plastisches Extrudat einem geeigneten Formkalander zugeführt. Geeignete Formkalander weisen zur Formung in der Regel Formwalzen und/oder Bänder auf, wobei mindestens eine der Formwalzen und/oder mindestens eines der Bänder Vertiefungen zur Aufnahme und Formung des plastischen Gemischs aufweist. Vorzugsweise verwendet man einen Formkalander mit gegenläufig rotierenden Formwalzen, wobei mindestens eine der Formwalzen auf ihrer Oberfläche Vertiefungen zur Aufnahme und Formung des plastischen Gemischs aufweist. Geeignete Formkalander und Formwalzen enthaltende Vorrichtungen sind allgemein beispielsweise in der EP-A-0 240 904, EP-A-0 240 906 und WO 96/19962, geeignete Bänder und Bänder enthaltende Vorrichtungen allgemein beispielsweise in der EP-A-0 358 105 offenbart, auf die hiermit Bezug genommen wird.

Die Formgebung des noch plastischen Gemischs oder noch plastischen Extrudats erfolgt vorzugsweise bei Temperaturen unterhalb von 220 °C, besonders bevorzugt unterhalb von 180 °C und ganz besonders bevorzugt unterhalb von 150 °C, wie z. B. in den zur Bildung des plastischen Gemischs notwendigen Temperaturbereichen oder bei niedrigeren Temperaturen. Wenn die Formung bei niedrigeren Temperaturen erfolgt, erfolgt sie vorteilhaft 5 bis 70 °C, bevorzugt 10 bis 50 °C und besonders bevorzugt 15 bis 40 °C unterhalb der höchsten bei der Bildung des plastischen Gemischs erreichten Temperatur, vorzugsweise jedoch oberhalb der Erstarrungstemperatur des plastischen Gemischs.

Weiterer Gegenstand der vorliegenden Erfindung ist eine feste, im Wesentlichen von aliphatischen C₂-C₈-Di- und Tricarbonsäuren und aromatischen C₆-C₁₀-Monocarbonsäuren freie Dosierungsform, die erhältlich ist durch ein wie zuvor beschriebenes Verfahren. Die Dosierungsform kann gegebenenfalls organische Säuren, wie aliphatische C₁-C₃₂-Monocarbonsäuren, Aminosäuren und Aminosäurederivate enthalten. Besonders bevorzugt ist die erfindungsgemäße Dosierungsform im Wesentlichen frei von Säuren.

Vorzugsweise liegen in den erfindungsgemäßen Dosierungsformen mindestens 10 Gew.-% des Wirkstoffs in Form eines Cyclodextrin-Wirkstoff-Komplexes vor.

Die nachfolgenden Beispiele sollen die Erfindung illustrieren ohne sie zu beschränken.

### Beispiele

Die Extrusion erfolgte jeweils mit einem Zweischneckenextruder ZKS-30 der Firma Werner und Pfleiderer mit fünf Schüssen unter den in dem jeweiligen Beispiel angegebenen Bedingungen.

### Vergleichsbeispiel 1

475 g Polyethylenglykol PEG 6000 wurden mit 25 g β-Estradiol extrudiert und zu 1 000 mg-Oblong-Tabletten kalandriert. Die Extrusion erfolgte unter folgenden Bedingungen:

| | |
|---|---|
| Schuss 1 | 20 °C |
| Schuss 2 | 40 °C |
| Schuss 3 | 50 °C |
| Schuss 4 | 60 °C |
| Schuss 5 | 50 °C |
| Düse | 42 °C |

Die Freisetzung des Wirkstoffs aus den Tabletten wurde nach dem Paddle-Modell (USP; 0,1 M Salzsäure; pH 1,0; 150 Upm) untersucht. Sie betrug nach 10 Minuten 8 % und nach 20 und 30 Minuten immer noch unter 10 %.

### Vergleichsbeispiel 2

Bei einer aus Vergleichsbeispiel 1 erhaltenen Dosierungsform wurde die Freisetzung des Wirkstoffs aus den Tabletten unter den analogen Bedingungen (USP; 0,1 M Salzsäure; pH 1,0; 150 Upm) bestimmt, jedoch wurde dem Prüfmedium zuvor 150 mg β-Cyclodextrin W7M1.8 (Fa. Wacker, methyliertes β-Cyclodextrin mit einem Methylierungsgrad von 1,8) zugesetzt. Die Freisetzung betrug nach 10 Minuten 8 %, nach 20 Minuten 24 % und nach 30 Minuten 29 %.

### Beispiel 1

400 g Polyethylenglykol PEG 6000 wurden mit 25 g β-Estradiol und 75 g β-Cyclodextrin W7M1.8 (Fa. Wacker) extrudiert und zu 1 000 mg-Oblong-Tabletten kalandriert.

Die Extrusion erfolgte unter folgenden Bedingungen:

| | |
|---|---|
| Schuss 1 | 20 °C |
| Schuss 2 | 40 °C |
| Schuss 3 | 50 °C |
| Schuss 4 | 60 °C |
| Schuss 5 | 50 °C |
| Düse | 42 °C |

Die Freisetzung des Wirkstoffs aus den Tabletten wurde nach dem Paddle-Modell (USP; 0,1 M Salzsäure; pH 1,0; 150 Upm) untersucht. Sie betrug nach 10 Minuten 48 %, nach 20 Minuten 77 % und nach 30 Minuten 80 %.

### Vergleichsbeispiel 3

400 g Polyethylenglykol PEG 6000 wurden mit 100 g Ibuprofen extrudiert und zu 1 000 mg-Oblong-Tabletten kalandriert.

Die Extrusion erfolgte unter folgenden Bedingungen:

| | |
|---|---|
| Schuss 1 | 20 °C |
| Schuss 2 | 40 °C |
| Schuss 3 | 50 °C |
| Schuss 4 | 60 °C |
| Schuss 5 | 50 °C |
| Düse | 42 °C |

Die Freisetzung des Wirkstoffs aus den Tabletten wurde nach dem Paddle-Modell (USP; 0,1 M Salzsäure; pH 1,0; 150 Upm) untersucht. Sie betrug nach 20 Minuten 6 %, nach 60 Minuten 5 % und nach 120 Minuten 7 %.

### Vergleichsbeispiel 4

Bei einer aus Vergleichsbeispiel 3 erhaltenen Dosierungsform wurde die Freisetzung des Wirkstoffs aus den Tabletten unter den analogen Bedingungen (USP; 0,1 M Salzsäure; pH 1,0; 150 Upm) bestimmt, jedoch wurde dem Prüfmedium zuvor 150 mg β-Cyclodextrin W7M1.8 zugesetzt. Die Freisetzung betrug nach 10 Minuten 8 %, nach 20 Minuten 24 % und nach 30 Minuten 29 %.

Die Freisetzung des Wirkstoffs aus den Tabletten wurde nach dem Paddle-Modell (USP; 0,1 M Salzsäure; pH 1,0; 150 Upm) untersucht. Sie betrug nach 20 Minuten 6 %, nach 60 Minuten 9 % und nach 120 Minuten 15 %.

### Beispiel 2

250 g polyethylenglykol PEG 6000 wurden mit 100 g Ibuprofen und 150 g β-Cyclodextrin W7M1.8 extrudiert und zu 1 000 mg-oblong-Tabletten kalandriert.

Die Extrusion erfolgte unter folgenden Bedingungen:

| | |
|---|---|
| Schuss 1 | 20 °C |
| Schuss 2 | 40 °C |
| Schuss 3 | 50 °C |
| Schuss 4 | 60 °C |
| Schuss 5 | 50 °C |
| Düse | 42 °C |

Die Freisetzung des Wirkstoffs aus den Tabletten wurde nach dem Paddle-Modell (USP; 0,1 M Salzsäure; pH 1,0; 150 Upm) untersucht. Sie betrug nach 20 Minuten 18 %, nach 60 Minuten 24 % und nach 120 Minuten 26 %.

## Patentansprüche

1. Verfahren zur Herstellung von festen Dosierungsformen zur oralen oder rektalen Verabreichung bei Mensch und Tier, wobei man
a) 0,5 bis 30 Gew.-% mindestens eines Wirkstoffs,
b) 0,5 bis 70 Gew.-% mindestens eines Cyclodextrins,
c) 10 bis 98 Gew.-% mindestens eines polymeren Bindemittels, das ausgewählt ist unter Polyethylenglykol mit einem Molekulargewicht oberhalb von 1000, Polyvinylpyrrolidon oder Copolymeren, enthaltend N-Vinylpyrrolidon und Vinylacetat,
d) 0 bis 50,0 Gew.-% übliche Hilfsmittel
bei einer Temperatur unterhalb von 220 °C ohne Zugabe eines Lösungsmittels vermischt und plastifiziert und das erhaltene plastische Gemisch zur Dosierungsform formt.

2. Verfahren nach Anspruch 1, **dadurch gekennzeichnet, dass** das Molverhältnis zwischen Wirkstoff und Cyclodextrin im Bereich von 0,1 bis 4,0 liegt.

3. Verfahren nach einem der vorhergehenden Ansprüche, **dadurch gekennzeichnet, dass** man das plastische Gemisch in einem Formkalander zu Dosierungsformen formt.

4. Verfahren nach Anspruch 3, **dadurch gekennzeichnet, dass** man einen Formkalander mit gegenläufig rotierenden Formwalzen verwendet, wobei mindestens eine der Formwalzen auf ihrer Oberfläche Vertiefungen zur Aufnahme und Formung des plastischen Gemisches aufweist.

5. Feste, von aliphatischen C₂-C₈-Di- und -Tricarbonsäuren und aromatischen C₆-C₁₀-Monocarbonsäuren freie Dosierungsform, erhältlich durch ein Verfahren gemäß einem der Ansprüche 1 bis 4.

6. Feste Dosierungsform nach Anspruch 5, **dadurch gekennzeichnet, dass** mindestens 10 Gew.-% des Wirkstoffs in Form eines Cyclodextrin-Wirkstoff-Komplexes vorliegen.

## Claims

1. A process for producing solid dosage forms for oral or rectal administration to humans and animals, wherein
a) 0.5 to 30% by weight of at least one active ingredient,
b) 0.5 to 70% by weight of at least one cyclodextrin,
c) 10 to 98% by weight of at least one polymeric binder selected from among polyethylene glycol having a molecular weight of greater than 1000, polyvinylpyrrolidone or copolymers comprising N-vinylpyrrolidone and vinyl acetate,
d) 0 to 50% by weight of conventional excipients.
are mixed and plasticized at a temperature below 220°C without adding a solvent, and the resulting plastic mixture is shaped to the dosage form.

2. A process as claimed in claim 1, wherein the molar ratio between active ingredient and cyclodextrin is in the range from 0.1 to 4.0.

3. A process as claimed in any of the preceding claims, wherein the plastic mixture is shaped in a molding calender to dosage forms.

4. A process as claimed in claim 3, wherein a molding calender with counterrotating molding rolls is used, with at least one of the molding rolls having on its surface depressions to receive and shape the plastic mixture.

5. A solid dosage form which is free of aliphatic C₂-C₈-di- and -tricarboxylic acids and aromatic C₆-C₁₀-monocarboxylic acids, obtainable by a process as claimed in any of claims 1 to 4.

6. A solid dosage form as claimed in claim 5, wherein at least 10% by weight of the active ingredient are present in the form of a cyclodextrin/active ingredient complex.

## Revendications

1. Procédé pour la préparation de formes de dosage solides pour l'administration orale ou rectale à des humains ou des animaux, selon lequel on mélange et on plastifie
a) 0,5 à 30 % en poids d'au moins une substance active,
b) 0,5 à 70 % en poids d'au moins une cyclodextrine,
c) 10 à 98 % en poids d'au moins un liant polymère choisi parmi les polyéthylèneglycols de poids moléculaire supérieur à 1000, la polyvinylpyrrolidone et les copolymères contenant de la N-vinylpyrrolidone et de l'acétate de vinyle,
d) 0 à 50,0 % en poids de produits auxiliaires usuels,
à une température inférieure à 220° C, sans adjonction de solvant, puis on met le mélange plastique ainsi obtenu sous la forme de dosage.

2. Procédé selon la revendication 1, **caractérisé par le fait que** le rapport molaire entre la substance active et la cyclodextrine se situe dans l'intervalle de 0,1 à 4,0.

3. Procédé selon l'une des revendications qui précèdent, **caractérisé par le fait que** le mélange plastique est mis à l'état de formes de dosage dans une calandre de façonnage.

4. Procédé selon la revendication 3, **caractérisé par le fait que** l'on utilise une calandre de façonnage à cylindres de façonnage tournant en sens inverse, l'un au moins des cylindres de façonnage portant sur sa surface des cavités qui recueillent et façonnent le mélange plastique.

5. Forme de dosage solide exempte d'acides dicarboxyliques et tricarboxyliques aliphatiques en C2-C8 et d'acides aromatiques monocarboxyliques en C6-C10, obtenue par un procédé selon l'une des revendications 1 à 4.

6. Forme de dosage solide selon la revendication 5, **caractérisée par le fait qu'**au moins 10 % en poids de la substance active sont à l'état de complexe cyclodextrine-substance active.
